# EUROPEAN PATENT APPLICATION

(11) **EP 4 649 891 A1**
(43) Date of publication of application: **19.11.2025**
(21) Application number: 25176305.8
(22) Date of filing: 14.05.2025
(51) Int. Cl.: A61B 8/00, G06T 7/00, A61B 8/06

(54) **MEDICAL INFORMATION PROCESSING APPARATUS AND MEDICAL INFORMATION PROCESSING METHOD**

(30) Priority: 14.05.2024 JP 2024078982; 09.05.2025 JP 2025078507
(71) Applicant: Canon Medical Systems Corporation, Tochigi 324-0036 (JP)
(72) Inventor: USUMURA, Masashi, Otawara-shi, 324-0036 (JP); IGARASHI, Seito, Otawara-shi, 324-0036 (JP)
(74) Representative: Marks & Clerk LLP

(57) **Abstract**

A medical information processing apparatus (100) includes an acquisition unit (111), a detection unit (112), a selection unit (113), and a synthesizing process unit (114). The acquisition unit (111) acquires a plurality of sets of ultrasonic data representing frames of a subject that are successive in a time direction. The detection unit (112) detects a motion of the subject. Based on a result of detecting the motion, the selection unit (113) selects a plurality of sets of frame data representing subjects to be synthesized from the sets of ultrasonic data. The synthesizing process unit (114) performs a synthesizing process on the selected sets of frame data.

## Description

### FIELD

Embodiments described herein relate generally to a medical information processing apparatus, an ultrasonic diagnostic apparatus, and a medical information processing method.

### BACKGROUND

A technique that improves image quality of ultrasonic images containing a microscopic passage and a fluid (for example, a blood flow or a contrast agent) has been known. For example, Patent Literature 1 discloses removing clutter contained in a reception signal of sequential ultrasonic in a given time and averaging the signal of a fluid after the removal of the clutter in a given time, and thereby generating an image presenting a border of a passage (for example, the shape of blood vessels) has been disclosed. Furthermore, Patent Literature 2 discloses performing super-resolution processing that increases a resolution (pixel density) of an ultrasonic image and sharpens a peak of a speckle pattern in the ultrasonic image, superimposing a plurality of speckle patterns with sharpened peaks in a given time, and thereby displaying a passage of a fluid in high image quality.

According to the above-described techniques, a larger number of frames to be used enable generation of a higher-quality image; however, the larger the number of frames to be used is, the longer a time of collecting frame data is, which increases a possibility of occurrence of artifact in an image because of occurrence of a body motion in a subject.

### SUMMARY OF INVENTION

A medical information processing apparatus provided in an aspect of the present embodiment includes an acquisition unit that acquires a plurality of sets of ultrasound data representing frames of a subject that are successive in a time direction, a detection unit that detects a motion of the subject, a selection unit that, based on a result of detecting the motion, selects a plurality of sets of frame data representing subjects to be synthesized from the sets of ultrasound data, and a synthesizing process unit that performs a synthesizing process on the selected sets of frame data.

The synthesizing process unit may add the sets of frame data and generates sum data.

The medical information processing apparatus may further comprise an extraction unit that executes a process for high definition of a subject that is presented by the sum data.

The medical information processing apparatus may further comprise an extraction unit that extracts signal components representing a subject from the sets of frame data, respectively, wherein the synthesizing process unit may perform a synthesizing process of generating integrated data obtained by integrating the signal components in the sets of frame data.

The extraction unit may generate filter information corresponding to each position in the frame data based on a result of extracting signal components representing the subject in the sets of frame data, and the synthesizing process unit may generate the integrated data using the filter information.

The medical information processing apparatus may further comprise an extraction unit that executes enhancing process of enhancing a subject on each of the sets of frame data, wherein the synthesizing process unit may add the sets of frame data after the enhancing process and generate sum data.

The detection unit may calculate an image quality evaluation index of each frame in the sets of ultrasound data and, based on the image quality evaluation index, detects a motion of the subject.

The detection unit may perform main component analysis on each frame in the sets of ultrasound data and, based on a result of the analysis, detects a motion of the subject.

The detection unit may detect a motion of the subject by inputting each frame in the sets of ultrasound data to a trained model that is trained using a data set including ultrasound data for training and information on presence or absence of a motion of a subject in the ultrasound data for training.

The selection unit may select a plurality of sets of frame data with relatively a few motions of the subject from the sets of ultrasound data as frame data representing frames that are the subjects to be synthesized.

The selection unit may select, as the frame data representing frames that are the subjects to be synthesized, frame data on a side of a larger number of frames from the ultrasound data of a set number of frames using a time at which a motion of the subject is detected by the detection unit as a border.

The selection unit may select, as the frame data representing frames that are the subjects to be synthesized, frame data that is acquired prior to a time at which a motion of the subject is detected by the detection unit in the sets of ultrasound data that are acquired over time.

The selection unit may select, as the frame data representing frames that are the subjects to be synthesized, a plurality of sets of frame data having similarity at or above a reference between the frames represented by the sets of ultrasound data.

An ultrasound diagnosis apparatus provided in an aspect of the present embodiment includes an execution unit that causes an ultrasound probe to execute ultrasound scanning, an acquisition unit that acquires a plurality of sets of ultrasound data representing frames of a subject that are successive in a time direction, a detection unit that detects a motion of the subject, a selection unit that, based on a result of detecting the motion, selects a plurality of sets of frame data representing subjects to be synthesized from the sets of ultrasound data, and a synthesizing process unit that performs a synthesizing process on the selected sets of frame data.

A medical information processing method provided in an aspect of the present embodiment includes an acquiring step of acquiring a plurality of sets of ultrasound data representing frames of a subject that are successive in a time direction, a detecting step of detecting a motion of the subject, a selecting step of, based on a result of detecting the motion, selecting a plurality of sets of frame data representing subjects to be synthesized from the sets of ultrasound data, and a synthesizing process of performing a synthesizing process on the selected sets of frame data.

### BRIEF DESCRIPTION OF THE DRAWINGS

FIG. 1 is a block diagram illustrating an example of a configuration of an ultrasonic diagnostic apparatus according to a first embodiment;
FIG. 2 is a flowchart illustrating a procedure of a process performed by the ultrasonic diagnostic apparatus according to the first embodiment;
FIG. 3 is a diagram for explaining detection of a motion according to the first embodiment;
FIG. 4A is a diagram illustrating an example of a selecting process according to the first embodiment;
FIG. 4B is a diagram illustrating an example of the selecting process according to the first embodiment;
FIG. 5 is a diagram for explaining an example of data processing according to the first embodiment;
FIG. 6 is a diagram for explaining an example of an extracting process according to the first embodiment;
FIG. 7 is a diagram illustrating an example of a result of a process according to a comparative example;
FIG. 8 is a diagram for explaining an example of data processing according to a second embodiment; and
FIG. 9 is a diagram for explaining an example of the data processing according to another embodiment.

### DETAILED DESCRIPTION

With reference to the accompanying drawings, a medical information processing apparatus, an ultrasonic diagnostic apparatus, and a medical information processing method according to the present application will be described in detail below. Note that the medical information processing apparatus, the ultrasonic diagnostic apparatus, and the medical information processing method according to the present application are not limited to the embodiments presented below.

### First Embodiment

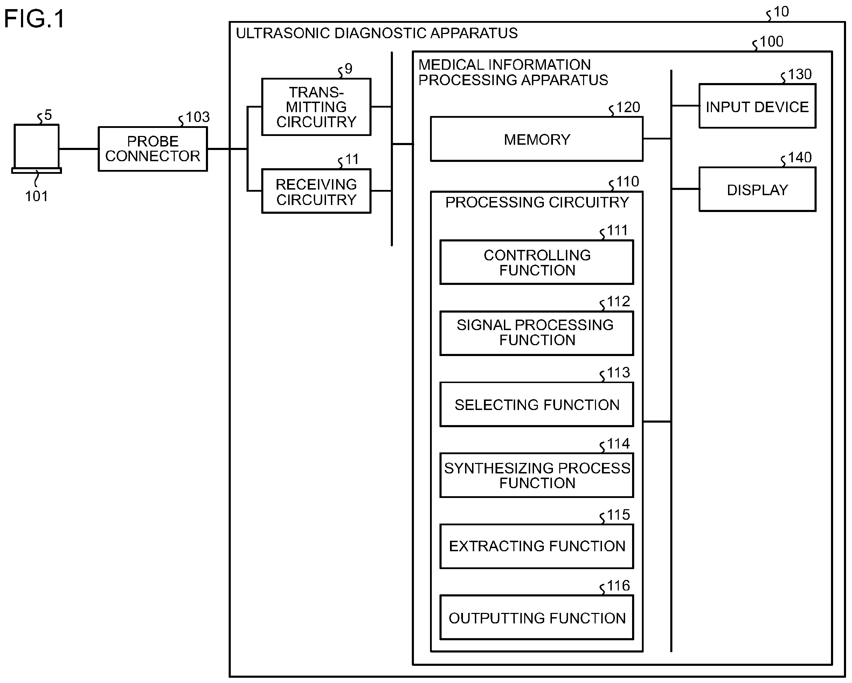

FIG. 1 is a block diagram illustrating an example of a configuration of an ultrasonic diagnostic apparatus 10 according to a first embodiment. The ultrasonic diagnostic apparatus 10 is an apparatus that generates ultrasonic data based on a reception signal (reflected-wave signal) that is received from an ultrasonic probe 5. The ultrasonic diagnostic apparatus 10 illustrated in FIG. 1 is an apparatus capable of generating two-dimensional ultrasonic data based on a reception signal received from the one-dimensional ultrasonic probe 5 (ultrasonic probe in which transducers are arrayed one-dimensionally) and generating three-dimensional ultrasonic data based on a reception signal received from the two-dimensional ultrasonic probe 5 (ultrasonic probe in which transducers are arrayed two-dimensionally).

The ultrasonic probe 5 is a probe of an electronic scanning system and has, at its tip, a plurality of transducers 101 that are arrayed one-dimensionally or two-dimensionally. The transducers 101 are piezoelectric elements (electromechanical transduction elements) that perform mutual transduction between an electric signal (voltage pulse signal) and ultrasonic (acoustic waves). The ultrasonic probe 5 transmits ultrasonic from the transducers 101 to a subject and receives the reflected ultrasonic from the subject with the transducers 101. Reflected acoustic waves reflect differences in acoustic impedance in the subject. Note that reflected acoustic waves in the case where transmitted ultrasonic pulses are reflected on a moving blood flow or a surface of the heart are subject to a frequency shift depending on speed signal components of a moving object with respect to an ultrasonic transmission direction because of the Doppler effect.

The ultrasonic probe 5 is connected to a probe connector 103 and the probe connector 103 transmits and receives ultrasonic to and from the ultrasonic probe 5. The probe connector 103 may connect the ultrasonic probe 5 in any one of a wired manner or a wireless manner. In a wired manner, the probe connector 103 includes a connector (receptacle) to which a connector (plug) of the ultrasonic probe 5 is connected. In a wireless manner, the probe connector 103 includes a communication unit that makes wireless communication with the ultrasonic probe 5.

The ultrasonic diagnostic apparatus 10 includes a transmitting circuit 9, a receiving circuit 11, and a medical information processing apparatus 100.

The transmitting circuit 9 is a transmitter that outputs a pulse signal (drive signal) to the transducers 101. The pulse signal is applied to the transducers 101 with time lags, accordingly ultrasonic of which delays are different is transmitted from the transducers 101, and accordingly a transmission ultrasonic beam is formed. Selectively changing the transducer 101 to which the pulse signal is applied (that is, the transducer 101 to be driven) and changing the delay of the pulse signal (application timing) make it possible to control the direction of the transmission ultrasonic beam and the focus. The direction and the fucus of the transmission ultrasonic beam are changed sequentially and accordingly an internal observation area of the subject is scanned. Furthermore, the delay of the pulse signal may be changed and accordingly a transmission ultrasonic beam that is a plane wave (of which focus is distant) or a diffusion wave (inverse to the ultrasonic transmission direction with respect to the transducers 101 with a polarity of focus points). Alternatively, a transmission ultrasonic beam may be formed using a single transducer or part of the transducers 101.

The transmitting circuit 9 transmits the pulse signal of a given drive waveform and accordingly the transducers 101 are caused to generate transmission ultrasonic having a given transmission waveform.

The receiving circuit 11 is a receiver to which an electric signal output from the transducer 101 that receives reflected ultrasonic is input as the reception signal. The reception signal is input to processing circuitry 110. In the first embodiment, an analog signal that is output from the transducer 101 and digital data obtained by sampling (performing digital conversion on) the analog signal are not particularly distinguished and are referred to as the reception signal.

The medical information processing apparatus 100 is connected to the transmitting circuit 9 and the receiving circuit 11 and executes processing on the signal that is received from the receiving circuit 11 and control on the transmitting circuit 9. The medical information processing apparatus 100 includes the processing circuitry 110, a memory 120, an input device 130, and a display 140.

The memory 120 includes a random access memory (RAM), a semiconductor memory device, such as a flash memory, a hard disk, and an optical disk. The memory 120 is a memory that stores data, such as image data for display that is generated by the processing circuitry 110. The memory 120 is also able to store the reception signal (reflected-wave signal) that is output by the receiving circuit 11. In addition to this, if required, the memory 120 stores various types of data, such as a control program for performing transmission and reception of ultrasonic, image processing, and display processing, diagnostic information (for example, a patient ID, an opinion of a doctor, or the like), a diagnostic protocol, and various types of body marks.

The input device 130 receives various types of instructions and information inputs from an operator. The input device 130 consists of, for example, a track ball, a switch button, a mouse, a keyboard, a touch pad via which an input operation is performed by touching an operational surface, a touch monitor that is integration of a display screen and a touch pad, a non-contact input circuit using an optical sensor, and an input interface device, such as an audio input circuit. Note that the input interface device is connected to the processing circuitry 110 described below, transduces an input operation that is received from the operator into an electric signal, and outputs the electric signal to the processing circuitry 110. Note that the input interface device herein is not limited to one including physical operational parts, such as a mouse and a keyboard. For example, examples of the input interface device include electric signal processing circuitry that receives an electric signal corresponding to an input operation from an external input device set independently of the device and that outputs the electric signal to the processing circuitry 110.

Under the control of the processing circuitry 110, the display 140 displays a graphical user interface (GUI) for receiving an input of an imaging condition and various types of images. The display 140 consists of, for example, a display interface device, sch as a liquid crystal display device.

By controlling each unit of the ultrasonic diagnostic apparatus 10, the processing circuitry 110 controls the entire ultrasonic diagnostic apparatus 10. For example, the processing circuitry 110 executes a program that is stored in the memory 120 and accordingly functions as a controlling function 111, a signal processing function 112, a selecting function 113, a synthesizing process function 114, an extracting function 115, and an outputting function 116. The processing circuitry 110 is realized using, for example, a processor. The controlling function 111 herein is an example of an execution unit and an acquisition unit. The signal processing function 112 is an example of a detection unit. The selecting function 113 is an example of a selection unit. The synthesizing process function 114 is an example of a synthesizing process unit. The extracting function 115 is an example of an extracting unit. Note that FIG. 1 illustrates the processing circuitry 110 as being realized as a single processor; however, the processing circuitry 110 may be realized using a large number of independent processors in combination. Alternatively, a specific function may be configured using a dedicated and independent circuit as in an application specific integrated circuit (ASIC).

The term "processor" used in the description above means, for example, a central processing unit (CPU), a graphical processing unit (GPU), or a circuit, such as an application specific integrated circuit (ASIC), a programmable logic device (for example, a simple programmable logic device (SPLD), a complex programmable logic device (CPLD), or a field programmable gate array (FPGA)). The processor reads programs that are saved in a memory 132 and executes the programs, thereby implementing the functions.

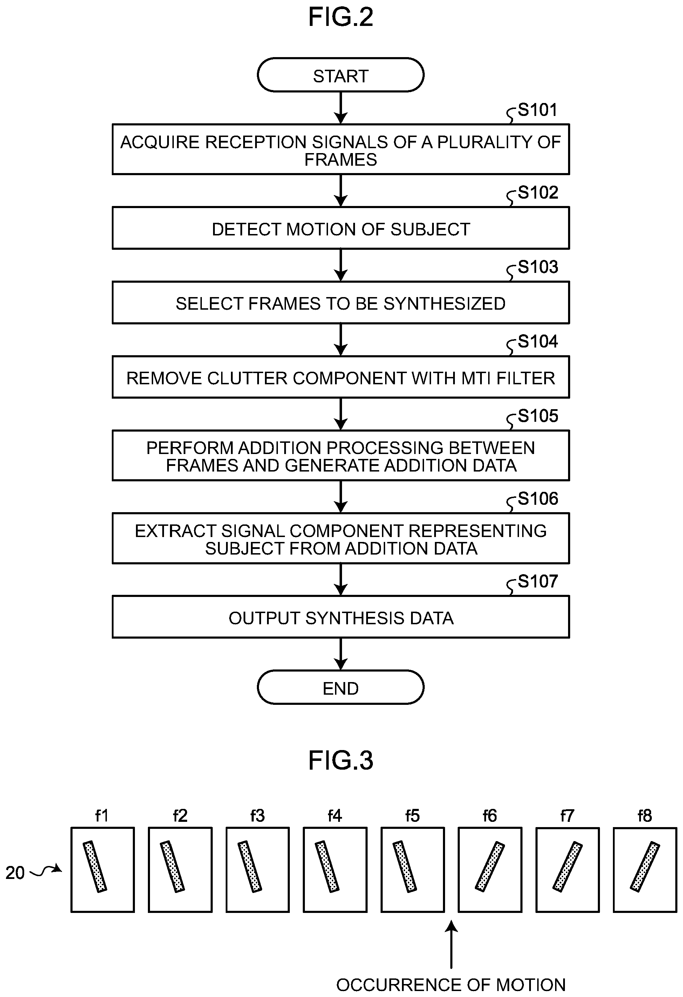

The ultrasonic diagnostic apparatus 10 configured as described above increases image quality of an ultrasonic image while avoiding occurrence of artifact caused by a body motion. Details of operations of the ultrasonic diagnostic apparatus 10 will be described according to FIG. 2 below. FIG. 2 is a flowchart illustrating a procedure of a process performed by the ultrasonic diagnostic apparatus 10 according to the first embodiment.

The controlling function 111 acquires a plurality of sets of ultrasonic data representing frames of a subject, respectively. Specifically, the controlling function 111 controls the transmitting circuit 9 and the receiving circuit 11, thereby causes the ultrasonic probe 5 to execute ultrasonic scanning, and acquires reception signals (for example, CH data) of the frames (step S101). More specifically, the controlling function 111 collects frame data (a plurality of sets of frame data in a given time) sequential in a time direction obtained by execution of ultrasonic scanning at a given frame rate. The frame data includes amplitude values (signal values) of signals corresponding to respective pixels forming one image (frame).

The signal processing function 112 performs phasing addition and quadrature detection on the collected reception signals. The phasing addition is processing of adding up reception signals of a plurality of the transducers 101 with a delay and a weight varied according to each transducer 101 and is also referred to as delay and sum (DAS) beam forming. Quadrature detection is processing of converting a reception signal into an in-phase signal and a quadrature signal of a baseband and acquiring IQ data and measurement data presenting an absolute value of the IQ data, and the like. Note that, in addition to this, processing using adaptive beam forming, model base processing, machine learning, and the like, may be performed on a reception signal. The signal processing function 112 also performs envelope detection processing, logarithmic compression processing, and the like, and thus generates B-mode data presenting a signal intensity in each point in an observation area by luminance.

The signal processing function 112 also detects a motion of the subject based on the sets of ultrasonic data (step S102). Specifically, the signal processing function 112 detects, in the sets of frame data acquired by the controlling function 111, frame data with a significant change in the position and orientation of the subject visualized in the image. For example, as illustrated in FIG. 3, the signal processing function 112 performs detection processing on frames f1 to f8 in frame data 20 and detects a frame f6 as frame data with a significant change in the position and orientation of the subject visualized in the image. In other words, the signal processing function 112 detects that a motion occurs in the subject between the frame f5 and the frame f6. Note that FIG. 3 is a diagram for explaining detection of a motion according to the first embodiment.

The signal processing function 112 is able to detect a motion of the subject by various methods. For example, the signal processing function 112 calculates an image quality evaluation index of each frame in the sets of ultrasonic data and detects a motion of the subject based on the calculated image quality evaluation index. In this case, the signal processing function 112 calculates an image quality evaluation index of each frame using an inter-frame difference, an optical flow, template matching, or the like, and detects a motion of the subject based on the calculated image quality indices.

For example, when the inter-frame difference is used, the signal processing function 112 calculates an inter-frame difference between frames that are adjacent temporally (or between a reference frame and a frame to be analyzed), thereby calculates amounts of motion in the images, respectively, and detects a frame with an amount of motion exceeding a threshold as a frame of occurrence of a motion in the subject.

For example, when the optical flow is used, the signal processing function 112 calculates a displacement vector representing a motion of the object between frames that are adjacent temporally (for example, elements having the same color) and specifies a transition of the amount of motion in a plurality of frames based on the calculated displacement vector. The signal processing function 112 extracts timing at which the amount of motion exceeds a threshold in the transition of the amount of motion and thereby detects a motion of the subject.

For example, when the template matching is used, the signal processing function 112 acquires a template image (for example, an image containing part of the subject) from a reference frame in the sets of frame data. Furthermore, the signal processing function 112 sets, in each frame, a search area (area in a given area with respect to an area of which template image is acquired) where a site similar to the template image is searched for. The signal processing function 112 performs template matching using the template image on the search area, calculates similarity to the template image in each position in the search area, and specifies a position having the highest similarity. The signal processing function 112 specifies the aforementioned position having the highest similarity in each frame and detects a frame in which the similarity in the specified position is under a threshold as a frame of occurrence of a motion in the subject.

For example, the signal processing function 112 is able to perform main component analysis on each frame in the sets of ultrasonic data and detect a motion of the subject based on the result of the analysis. In this case, the signal processing function 112 performs the main component analysis on each frame and thereby analyzes characteristics of the frames, respectively. The signal processing function 112 detects a frame of occurrence of a significant change in the analysis result as a frame of occurrence of a motion in the subject.

For example, the signal processing function 112 inputs each frame in the sets of ultrasonic data to a trained model that is trained using a data set including ultrasonic data for training and information on presence or absence of a motion of the subject in the ultrasonic data for training and thereby detects a motion of the subject. In this case, first of all, a trained model that is trained using, as data for learning, a plurality of sets of ultrasonic data containing a fine passage and a fluid (for example, a blood flow, a contrast agent, or the like) and presence or absence of a motion of the subject in the sets of ultrasonic data is prepared.

For example, because of training using a large number of frame data sets of frame data without occurrence of a motion between adjacent sets of frame data and sets of frame data with occurrence of a motion between sets of frame data, a trained model that outputs presence or absence of occurrence of a motion according to an input of the sets of frame data is prepared. The signal processing function 112 inputs the sets of frame data that are acquired by the controlling function 111 to the trained model and thereby detects a motion of the subject.

As described above, the signal processing function 112 detects a motion of the subject by various methods; however, the method of detecting a motion of the subject is not limited to the above-described methods. In other words, any method enabling detection of a motion of a subject may be used.

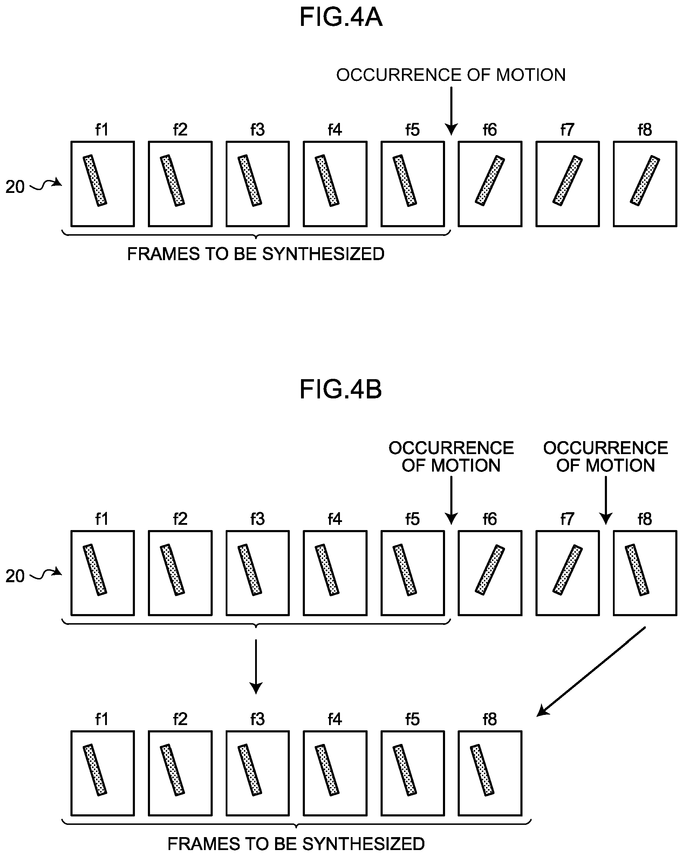

Based on the result of detecting a motion of the subject, the selecting function 113 selects frame data representing frames to be synthesized from the sets of ultrasonic data (step S103). Specifically, the selecting function 113 selects a plurality of sets of frame data with relatively a few motions of the subject from the sets of ultrasonic data as frame data representing frames to be synthesized. For example, as illustrated in FIG. 4A, the selecting function 113 selects frames f1 to f5 as frames to be synthesized from the frames f1 to f8. Note that FIG. 4A is a diagram illustrating an example of a selecting process according to the first embodiment.

The selecting function 113 is able to perform various selecting processes according to a situation in which high-quality image is generated. Specifically, the selecting function 113 executes different selecting processes respectively in the case of generating a high-quality image after ultrasonic scanning ends, in the case of, during ultrasonic scanning, generating high-quality image after ultrasonic data of a set number of frames is acquired, and in the case of generating a high-quality image while acquiring ultrasonic data by ultrasonic scanning.

In the case of generating a high-quality image by extracting signals from a plurality of sets of frame data and synthesizing the signals, a larger number of frames that are used enables generation of an image in higher quality. Thus, in the case of generating a high-quality image after ultrasonic scanning ends or in the case of, during ultrasonic scanning, generating a high-quality image after ultrasonic data of a determined number of frames is acquired, the selecting function 113 selects, as frame data representing frames to be synthesized, frame data on the side of a larger number of frames from the ultrasonic data of a set number of frames using a time at which a motion of the subject is detected by the signal processing function 112 as a border.

For example, as illustrated in FIG. 4A, in the case where one set for generating a high-quality image is set at eight frames, when the signal processing function 112 detects occurrence of a motion between the frame f5 and the frame f6, the selecting function 113 uses the time of occurrence of a motion serving as the border and selects frames f1 to f5 that are a larger number of frames as frames to be synthesized. Note that, if occurrence of a motion is detected between the frame f2 and the frame f3 in FIG. 4A, the selecting function 113 selects frames f3 to f8 as frames to be synthesized. When no motion of the subject is detected in the frames f1 to f8, the selecting function 113 selects the frames f1 to f8 as frames to be synthesized. Note that FIG. 4A illustrates the case where, for convenience of description, eight frames are set as the number of frames for generating a high-quality image; however, practically, few tens to one hundred is set as the number of frames (the number of packets).

On the other hand, when generating a high-quality image while acquiring ultrasonic data by ultrasonic scanning, the selecting function 113 selects, as frame data representing frames to be synthesized, frame data that is acquired prior to a time at which a motion of the subject is detected by the signal processing function 112 in a plurality of sets of ultrasonic data that are acquired over time.

For example, when the frame data 20 illustrated in FIG. 4A is acquired sequentially from the frame f1, the signal processing function 112 determines whether the subject has a motion each time frame data is acquired. In other words, after the frame f1 is acquired, the signal processing function 112 determines whether there is a motion of the subject each time frame data of the frame f2 or a subsequent frame is acquired. According to FIG. 4A, because occurrence of a motion is detected in the frame f6, the selecting function 113 selects the frames f1 to f5 before the detection of occurrence of a motion as frames to be synthesized.

Note that, when no motion of the subject is detected from the frame f1 to the frame f8 in FIG. 4A, the selecting function 113 selects the frames f1 to f8 as frames to be synthesized at the time when the acquired frames reach eight frames that is the set number of frames (in other words, at the time when the frame f8 is acquired).

As illustrated in FIG. 4A, when occurrence of a motion is detected between the frame f5 and the frame f6, the signal processing function 112 uses the frame f6 as a reference and detects whether there is a motion in the following frames (the frame f7, the frame f8 and a frame f9 and the following frames that are not illustrated in the drawing). The selecting function 113 selects frames to be synthesized by performing the above-described selecting process on the frame f6 and the following frames.

As described above, a larger number of frames to be synthesized enables generation of an image in higher quality. The selecting function 113 thus uses as many frames as possible as frame data to be synthesized, which makes it possible to select a plurality of sets of frame data having similarity at or above a reference between frames represented by the sets of ultrasonic data as frame data representing frames to be synthesized.

For example, as illustrated in FIG. 4B, there is a possibility that, after a motion occurs between the frame f5 and the frame f6, a motion will occur again between the frame f7 and the frame f8 and a state in which the object is visualized in the frame f8 will return to a state similar to one in which the object is visualized in frame f5. In such a case, in order to use as many frames as possible as frame data to be synthesized, the selecting function 113 calculates similarity between the sets of frame data and performs the selecting process such that frame data of which calculated similarity is at or above the threshold is contained in framed data to be synthesized. The similarity between sets of frame data is calculated using a known similarity calculation method as appropriate. Note that FIG. 4B is a diagram illustrating an example of the selecting process according to the first embodiment.

The signal processing function 112 generates blood-flow data obtained by extracting information derived from a blood flow in the measure data. For example, the signal processing function 112 applies a MTI (Moving Target Indicator) filter on the selected sets of frame data to be synthesized (step S104). Accordingly, information derived from tissue that is still between frames or tissue with little motion (tissue signal component (clutter)) is reduced and information derived from the blood flow (blood flow signal components) is extracted. A filter of which filter information is fixed, such as a Butterworth IIR (Infinite Impulse Response) filter or a polynomial regression filter, may be used as the MTI filter. The MTI filter may be an adaptive filter that changes a coefficient according to an input signal using eigendecomposition or singular value decomposition.

The signal processing function 112 is able to decompose the frame data into a plurality of bases by eigendecomposition or singular value decomposition and take out a specific base and thereby remove information derived from tissue and extract information derived from a blood flow. A Doppler processing function 110i enables the signal processing function 112 to calculate a velocity vector of each set of coordinates in reception signal data using a method, such as a vector Doppler method, speckle tracking, or vector flow mapping and also calculate a blood flow vector representing a magnitude and a direction of a blood flow. It is possible to employ, in addition to the exemplified methods, a method of extracting information derived from a blood flow contained in frame data (measurement data) or remove information derived from tissue.

Note that the signal processing function 112 is also able to estimate an amount of displacement of the subject between a plurality of sets of frame data to be synthesized and, based on the result of the estimation, correct each of the sets of frame data to be synthesized. Specifically, the signal processing function 112 calculates an amount of displacement of the subject between frames from the sets of frame data to be synthesized and corrects frame data based on the calculated amount of displacement.

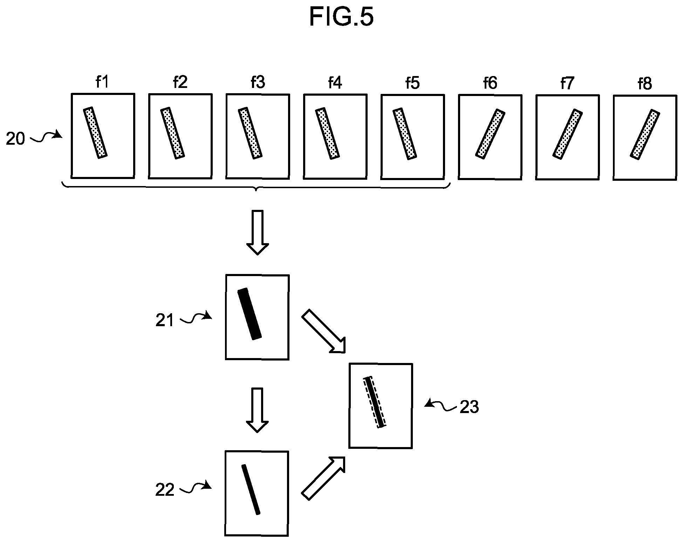

The synthesizing process function 114 performs a synthesizing process on the selected sets of frame data. Specifically, the synthesizing process function 114 generates addition data obtained by performing addition processing of a plurality of frames represented by the frame data (step S105). For example, as illustrated in FIG. 5, the synthesizing process function 114 generates addition data 21 by performing addition on a plurality of sets of frame data 20 (the frames f1 to f5) to be synthesized that are selected by the process at step S103 and from which clutter components are removed by the process at step S104. The synthesizing process function 114 generates addition data sequentially from the sets of frame data to be synthesized that are selected sequentially. Note that FIG. 5 is a diagram for explaining an example of data processing according to the first embodiment.

The addition data 21 has a signal-noise ratio (SN ratio) higher than that of each of the sets of frame data 20 before addition. The addition data 21 is, for example, blood-flow data (power Doppler data) in which information derived from the blood flow is enhanced. In the addition data 21, the signal of the fluid after removal of clutter is averaged in a given time and the addition data 21 presents a border of a passage. The frame represented by the addition data 21 contains a signal value (amplitude value) presenting a speckle pattern caused by enhancing interference and attenuating interference between ultrasonic reflected from the fluid and a signal value representing other noise (such as artifact) that occurs instantaneously or intermittently on a time axis.

The extracting function 115 extracts a signal component representing the subject from the addition data (step S106). Specifically, the extracting function 115 extracts a signal component representing the subject successively with respect to each of sets of addition data that are generated sequentially. The subject here has a high resolution and, for example, contains at least any one of blood, body tissue, and a contrast agent. Extracting a signal component representing the object successively means extracting a position of a signal (pixel) representing the object without given intervals in a signal space or an image space. For example, when the subject is blood, the extracting function 115 extracts a position of a signal (pixel) representing blood in the signal space or the image space without given intervals.

For example, as illustrated in FIG. 5, the extracting function 115 executes the extracting process for extracting a signal component representing the subject successively on the addition data 21 generated by the synthesizing process function 114, thereby acquiring a signal component 22 that is extracted data representing the subject.

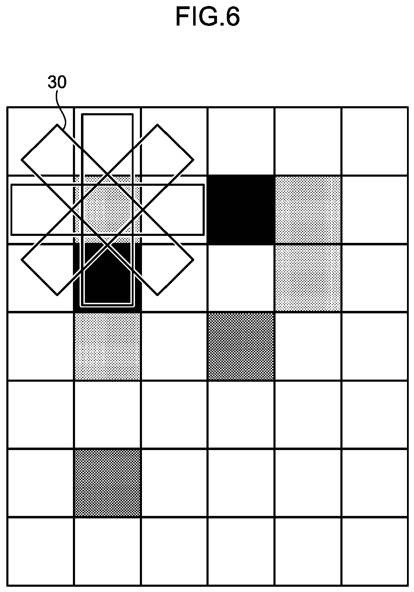

FIG. 6 is a diagram for explaining an example of the extracting process according to the first embodiment. As illustrated in FIG. 6, the extracting function 115 extracts a position of a signal (pixel) representing the subject (for example, blood) using a kernel 30. Specifically, the extracting function 115 arranges the kernel 30 in a position of interest in the frame (image) represented by the addition data 21, compares a pixel value (luminance) corresponding to the position of interest and a pixel value (luminance) corresponding to a position of a pixel in an area (around the position of interest) in which the kernel 30 is arranged, and extracts a position of a signal representing the position of the subject.

For example, as illustrated in FIG. 6, in the case where the kernel 30 based on the ratio of vertical 3×horizontal 1, the extracting function 115 arranges the center (the second cell) of the kernel 30 in the position of interest and, when a pixel value corresponding to that position is larger than or equal to pixel values corresponding to positions of both ends (the first and third cells) of the kernel 30, extracts a signal component of the position of interest as a signal component representing the subject. The extracting function 115 sets each pixel of the addition data 21 for the position of interest and executes the above-described extracting process sequentially. In other words, the extracting function 115 determines whether a signal component is contained with respect to all the pixels contained in the addition data 21. In this manner, the extracting function 115 extracts a signal component representing the subject successively.

FIG. 6 illustrates the example where the kernel 30 is arranged in four directions that are vertical, horizontal, diagonally upper right, and diagonally upper left directions, and thereby extracts a signal component representing the subject; however, the shape and the size of the kernel are not limited to this. FIG. 6 illustrates the example where the subject is extracted on a pixel-by-pixel basis; however, a signal component may be extracted with respect to each position of the frame based on each group including a plurality of pixels. In the case where a signal component is extracted based on each group including a plurality of pixels, an average or an integrated value of signal values of respective positions forming a group may serve as a pixel value (luminance) corresponding to each position of the frame.

The outputting function 116 outputs synthesis data based on the signal components that are extracted by the extracting function 115 and the addition data (S107). For example, as illustrated in FIG. 5, the outputting function 116 outputs synthesis data 23 based on the addition data 21 and the signal component 22. The outputting function 116 performs the synthesizing process and a correcting process on the signal component 22 and the addition data 21, thereby generating the synthesis data 23. The synthesizing process is a process of synthesizing (addition processing) the signal component 22 (extracted data) and the addition data 21 (power signal data). The correcting process is a process of correcting the addition data 21 (power signal data) based on the signal component 22 (extracted data).

As described above, by adjusting the number of frames (the number of frames to be synthesized) that are used for analysis in accordance with presence or absence of a motion in each frame, the ultrasonic diagnostic apparatus 10 is able to increase image quality of an ultrasonic image while avoiding occurrence of artifact caused by a body motion even when a motion occurs in the subject.

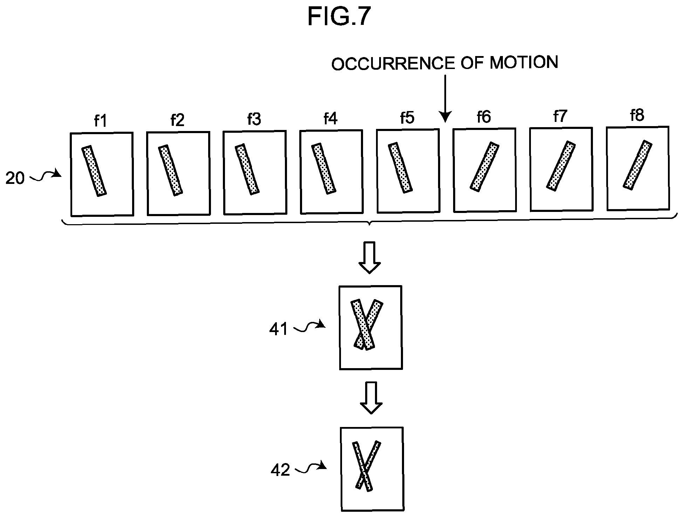

FIG. 7 is a diagram illustrating an example of a result of the process according to a comparative example. FIG. 7 illustrates the case where the number of frames that are used for analysis is not adjusted in accordance with presence or absence of a motion in each frame. As illustrated in FIG. 7, in the case where the synthesizing process is performed using, as frame data to be synthesized, eight frames (frames f1 to f8) in which a motion of the subject occurs between the frame f5 and the frame f6, the motion of the subject is visualized as artifact in generated addition data 41. Furthermore, when signal component extraction is performed on the addition data 41, a signal component 42 containing artifact is extracted as illustrated in FIG. 7.

As described above, according to the first embodiment, the controlling function 111 acquires a plurality of sets of ultrasonic data representing frames of the subject, respectively. Based on the sets of ultrasonic data, the signal processing function 112 detects a motion of the subject. Based on the result of detecting a motion of the subject, the selecting function 113 selects frame data representing frames to be synthesized from the sets of ultrasonic data. The synthesizing process function 114 performs the synthesizing process on the selected frame data. Accordingly, the ultrasonic diagnostic apparatus 10 according to the first embodiment is able to selectively change the frame data to be synthesized according to a motion of the subject and enables an increase in image quality of an ultrasonic image while avoiding occurrence of artifact caused by a body motion.

According to the first embodiment, the synthesizing process function 114 performs the synthesizing process of generating addition data obtained by performing addition processing of a plurality of frames represented by frame data. The extracting function 115 extracts a signal component representing the subject from the addition data. Accordingly, the ultrasonic diagnostic apparatus 10 according to the first embodiment enables generation of a high-quality image not containing artifact.

According to the first embodiment, the signal processing function 112 calculates an image quality evaluation index of each frame in the sets of ultrasonic data and detects a motion of the subject based on the calculated image quality evaluation index. The signal processing function 112 also performs the main component analysis on each frame in the sets of ultrasonic data and detects a motion of the subject based on the result of the analysis. The signal processing function 112 also inputs each frame in the sets of ultrasonic data to a trained model that is trained using a data set including ultrasonic data for training and information on presence or absence of a motion of the subject in the ultrasonic data for training and thereby detects a motion of the subject. Accordingly, the ultrasonic diagnostic apparatus 10 according to the first embodiment is able to detect a motion of the subject by various methods.

According to the first embodiment, the selecting function 113 selects a plurality of sets of frame data with relatively a few motions of the subject from the sets of ultrasonic data as frame data representing frames to be synthesized. Accordingly, the ultrasonic diagnostic apparatus 10 according to the first embodiment is able to select frame data containing no motion of the subject.

According to the first embodiment, the selecting function 113 selects, as frame data representing frames to be synthesized, frame data on the side of a larger number of frames from the ultrasonic data of a set number of frames using a time at which a motion of the subject is detected by the signal processing function 112 as a border. The selecting function 113 selects, as frame data representing frames to be synthesized, frame data that is acquired prior to a time at which a motion of the subject is detected by the signal processing function 112 in a plurality of sets of ultrasonic data that are acquired over time. The selecting function 113 selects a plurality of sets of frame data having similarity at or above a reference between frames represented by the sets of ultrasonic data as frame data representing frames to be synthesized. Thus, the ultrasonic diagnostic apparatus 10 according to the first embodiment is able to performs the synthesizing process using as much frame data as possible and, even when a motion occurs in the subject, generate an image in higher quality.

### Second Embodiment

In the first embodiment described above, the case where a signal component representing a subject is extracted from addition data obtained by executing addition processing on a plurality of sets of frame data to be synthesized is described. In a second embodiment, the case where signal components representing a subject are extracted respectively from a plurality of sets of frame data to be synthesized and the synthesizing process is performed on each of the extracted signal components will be described. Note that, in the second embodiment, compared to the first embodiment, the content of the process performed by the synthesizing process function 114 and the content of the process performed by the extracting function 115 are different. The processes will be described mainly below.

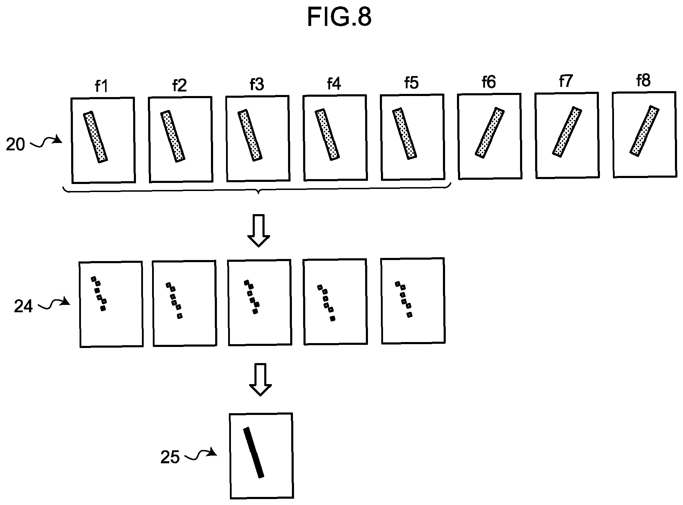

The extracting function 115 according to the second embodiment extracts signal components representing a subject respectively from a plurality of frames represented by frame data. Specifically, the extracting function 115 executes a process of extracting a signal component on each of a plurality of sets of frame data to be synthesized that are selected by the selecting function 113. FIG. 8 is a diagram for explaining an example of data processing according to the second embodiment. FIG. 8 illustrates the case where a motion of the subject is detected between a frame f5 and a frame f6 in the frame data 20 and frames f1 to f5 are selected as sets of frame data to be synthesized.

As illustrated in FIG. 8, the extracting function 115 executes an extracting process on the frames f1, f2, f3, f4, and f5 to be synthesized and thereby extracts a signal component 24 corresponding to each frame.

The synthesizing process function 114 according to the second embodiment performs the synthesizing process of generating integrated data obtained by integrating the signal components of the respective frames. Specifically, the synthesizing process function 114 generates integrated data obtained by integrating a plurality of signal components that are extracted by the extracting function 115 respectively from the sets of frame data to be synthesized. For example, as illustrated in FIG. 8, the synthesizing process function 114 generates integrated data 25 by integrating five signal components 24 that are extracted from the frames f1 to f5, respectively.

The above-described example illustrates the case where signal components are extracted from sets of frame data to be synthesized that are selected along a result of detecting a motion of the subject; however, embodiments are not limited to this, and a motion of the subject may be detected after signal components are extracted. In such a case, for example, the extracting function 115 extracts signal components representing a subject from all the frames f1 to f8 illustrated in FIG. 8. The signal processing function 112 detects a motion of the subject based on the extracted signal components and, based on the result of detecting a motion, the selecting function 113 selects a plurality of signal components to be synthesized. The synthesizing process function 114 generates integrated data obtained by integrating the signal components to be synthesized.

As described above, according to the second embodiment, the extracting function 115 extracts respective signal components representing a subject from a plurality of frames represented by sets of frame data. The synthesizing process function 114 performs the synthesizing process of generating integrated data obtained by integrating the respective signal components of the frames. Accordingly, the ultrasonic diagnostic apparatus 10 according to the second embodiment is able to extract signal components from frame data containing no effect of a body motion and enables generation of a high-quality image containing no artifact.

### Another Embodiment

In addition to the above-described embodiments, the ultrasonic diagnostic apparatus 10 according to the present application is able to acquire information on whether a signal component is extracted in each position in a frame in a plurality of frames that are selected as sets of frame data to be synthesized and acquire filter information obtained by converting the acquired information into a weight. Specifically, the extracting function 115 generates filter information corresponding to each position in frame data based on the result of extracting signal components representing a subject in a plurality of sets of frame data and the synthesizing process function 114 generates integrated data (or addition data) using the filter information. For example, the extracting function 115 acquires presence or absence of a signal component in each position in an image with respect to each of the frames to be synthesized (the frames f1 to f5) illustrated in FIG. 4A.
The extracting function 115 then generates filter information in which the larger the number of frames in which a signal component is extracted is, the higher the weight is with respect to the positions in the image. By using the aforementioned filter information when performing the synthesizing process, the synthesizing process function 114 generates addition data (or integrated data) where a position with higher frequency of extraction of a signal component is more displayed as a pixel representing the subject.

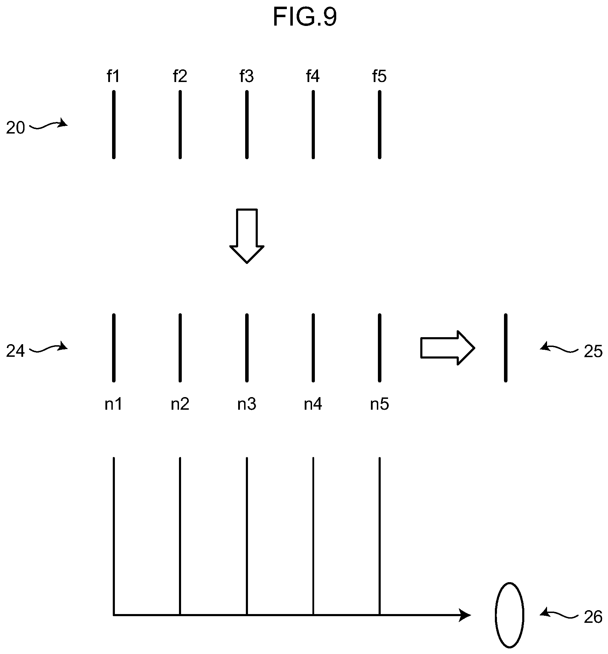

FIG. 9 is a diagram for explaining an example of data processing according to another embodiment. FIG. 9 illustrates, as an example, the case where, as illustrated in FIG. 8, the frames f1 to f5 in the frame data 20 are selected as sets of frame data to be synthesized. For example, as illustrated in FIG. 9, the extracting function 115 executes the above-described extracting process on the frames f1 to f5 to be synthesized and thereby extracts the signal component 24 corresponding to the subject with respect to each of the frames. The extracting process that is executed by the extracting function 115 is a process of identifying whether a pixel value in each position of the frame is larger than pixel values corresponding to the surrounding positions of the position.

By executing the above-described extracting process, the extracting function 115 according to the another embodiment acquires identifying information that identifies whether each position in the frame data to be synthesized has a signal value larger than signal values corresponding to the surrounding positions of the position and generates filter information based on the acquired identifying information. For example, as illustrated in FIG. 9, the extracting function 115 acquires filter information 26 from identifying information n1 based on the signal component 24 extracted from the frame f1, identifying information n2 based on the signal component 24 extracted from the frame f2, identifying information n3 based on the signal component 24 extracted from the frame f3, identifying information n4 based on the signal component 24 extracted from the frame f4, and identifying information n5 based on the signal component 24 extracted from the frame f5.

For example, in the case where the kernel illustrated in FIG. 6 (kernel based on the ratio of vertical 3×horizontal 1) is used, the extracting function 115 arranges the center (the second cell) of the kernel 30 in a position of interest, when a pixel value corresponding to that position is larger than (or equal to) pixel values corresponding to positions of both ends (the first and third cells) of the kernel 30, represents the position of interest by "1" and, when a pixel value corresponding to the position of interest is smaller than pixel values corresponding to positions around the position, acquires identifying information representing the position of interest by "0". By performing the process on each position in the frames f1 to f5, the extracting function 115 acquires sets of identifying information n1 to n5 on the respective sets of frame data. In other words, the extracting function 115 acquires a binary image obtained by converting each pixel into "0" or "1" with respect to each set of frame data.

Furthermore, based on the identifying information on the acquired five frames (sets of identifying information n1 to n5), the extracting function 115 acquires the filter information 26 corresponding to each position in the frame. The filter information 26 is calculated according to valued represented by the respective sets of identifying information (n1 to n5) on mutually corresponding positions (coordinates (x,y)) between the frames. For example, when mutually corresponding positions in the respective sets of identifying information have "1" in all the five frames, the filter information on the position is "1". When a corresponding position has "1" for three times and has "0" twice over the five frames, the filter information on the position is "0.6". When mutually corresponding positions have "1" in all the five frames, the filter information on the position is "0".

As described above, as for the sets of identifying information on the sets of frame data to be synthesized, when a corresponding position has "1" for a larger number of times, a larger value is set for the filter information 26. In other words, when a corresponding position has "1" for a larger number of times, in that position, a signal value larger than those of the surrounding positions is obtained successively and a fluid is highly likely present and therefore a weight coefficient serving as the filter information is set at a higher value for such a position. On the other hand, when "0" is contained in a corresponding position in each set of identifying information, noise (such as artifact) that occurs instantaneously or intermittently on a time axis is highly likely present in the position and therefore a weight coefficient serving as the filter information is set at a lower value for such a position.

The synthesizing process function 114 generates integrated data (or addition data) using the filter information 26. For example, as illustrated in FIG. 9, the synthesizing process function 114 generates the integrated data 25 by integrating the five signal components 24 that are extracted respectively from the frames f1 to f5. The synthesizing process function 114 then generates integrated data using the filter information 26 by multiplying the pixel value representing each position in the generated integrated data 25 by the filter information (weight coefficient) that is set in each corresponding position in the filter information 26. In such integrated data, using the filter information 26, a position where a fluid is highly likely present in the frames to be synthesized is multiplied by a weight coefficient that is a large value and thus the position of the fluid is enhanced. On the other hand, in such integrated data, using the filter information 26, a position where noise (such as artifact) is highly likely present in the frames to be synthesized is multiplied by a weight coefficient that is a small value and thus noise is reduced.

In the second embodiment, weight information that is determined according to each of the sets of frame data may be applied to signal components that are extracted by the extracting function 115 from the sets of frame data, respectively. For example, with respect to each of the sets of frame data, for frame data with an elapse of shorter time, weight information that is a higher value may be applied to a signal component that is extracted from the frame data. Accordingly, it is possible to weight a signal component that is extracted from each of the sets of frame data according to the elapse of time.

In the first embodiment, the example where the extracting function 115 uses the kernel 30 based on the ratio of vertical 3×horizontal 1, arranges the kernel 30 in four directions that are vertical, horizontal, diagonally upper right, and diagonally upper left directions, and, with respect to each position in the frame, extracts a signal component based on comparison between a pixel value corresponding to the position and pixel values corresponding to surrounding positions in the respective directions and within a distance has been described; however, embodiments are not limited to this. The surrounding positions of which pixel values are compared by the extracting function 115 may be positions in a given direction and within a given distance with respect to each position in the frame. The extracting function 115 may apply weight information that is determined according to at least any one of the directions and the distance to the signal component.

In the above-described embodiment, the case where the extracting function 115 executes the process for extracting process of extracting a signal component representing a subject has been described as a process for high resolution of a subject. As for the process for high resolution of a subject, however, a process other than the above-described process may be performed. For example, the extracting function 115 may enable high resolution of a subject by performing peak sharpening that applies a non-linear function to a pixel value in a frame. In such a case, for example, first of all, the extracting function 115 performs a process of performing resampling on the addition data 21 illustrated in FIG. 5 and thereby increasing a pixel density and increasing an effective resolution. Resampling, for example, replaces each pixel with a plurality of pixels in smaller size. The signal value of each pixel after replacement may be interpolated from an original signal value (signal value before replacement) by, for example, bicubic interpolation, or the like.

Furthermore, by performing exponentiation (for example, to the power of 8 or 12) on each pixel value of the addition data 21 after resampling, the extracting function 115 sharpens the pixel value (signal component relatively larger than those of surrounding pixels) representing a subject (a pixel value representing the subject is sometimes referred to as a local peak below). The extracting function 115 extracts a position of a local peak by performing, for example, thresholding, or the like, on the pixel value after exponentiation and thereby extracts a position of the local peak. Note that the subject of the aforementioned processing of sharpening the local peak is not limited to addition data and may be frame data. In other words, the extracting function 115 executes an enhancing process of enhancing a subject on each of the sets of frame data and the synthesizing process function 114 generates addition data by performing addition processing of the sets of frame data after the enhancing process. For example, the extracting function 115 executes the above-described processing of sharpening the local peak on each of the frames f1 to f5 illustrated in FIG. 8. The synthesizing process function 114 adds the sets of frame data after the processing of sharpening the local peak and thereby generates addition data.

In the above-described embodiment, the example where the synthesizing process function 114 performs addition processing on a plurality of sets of frame data to be synthesized and thereby generates single set of addition data whose SN ratio is increased has been described; however, ultrasonic data whose SN ratio is increased without addition processing may be acquired. In such a case, for example, the synthesizing process function 114 may input a plurality of sets of frame data 20 to be synthesized to a trained model to which a plurality of sets of frame data sequential in a time direction and obtained by execution of ultrasonic scanning is input and that thus outputs a single set of ultrasonic data whose SN ratio is higher than the frame data and may acquire the single set of ultrasonic data that is output from the trained model as the addition data 21. The trained model is trained using a data set including a plurality of sets of frame data as input data and using a single set of ultrasonic data whose SN ratio is higher than that of each of the sets of frame data as training data, or the like. The synthesizing process function 114 inputs the sets of the frame data 20 to the trained model and acquires, as the addition data 21, the single set of ultrasonic data whose SN is ratio is higher than that of the sets of frame data and that is output from the trained model.

In the above-described first embodiment, the example of application to an ultrasonic diagnostic apparatus has been described; however, embodiments are not limited to this, and application to a medical information processing apparatus other than ultrasonic diagnostic apparatus may be made. For example, application to a medical information processing apparatus, such as a work station or a server that acquires ultrasonic data that is obtained based on a result of ultrasonic scanning on a subject, can be made. For example, a medical information processing apparatus, such as a work station or a server, may execute the above-described process using a plurality of sets of frame data that were collected in the past.

In the description above, the fluid presented by the ultrasonic image is described exemplifying a blood flow as an example and thus the example where the controlling function 111 acquires first ultrasonic data obtained by ultrasonic scanning without presence of a contrast agent has been described; however, the fluid may be a contrast agent. In that case, the controlling function 111 may acquire a plurality of sets of ultrasonic data obtained by ultrasonic scanning in the presence of a contrast agent.

Each of the components of each of the apparatuses illustrated in the drawings in the description of the above-described embodiments is of functional concepts and need not necessarily be configured physically as illustrated in the drawings. In other words, specific modes of distribution and integration of each apparatus are not limited to those illustrated in the drawings, and all or part of the apparatuses may be configured by being distributed or integrated functionally or physically in any unit according to various types of load and usage. Furthermore, all or part of each processing function implemented in each apparatus can be implemented by a CPU and using a program that is analyzed and implemented by the CPU or can be implemented as hardware using wired logic.

The methods described in the above-described embodiments can be implemented by executing a program that is prepared in advance using a computer, such as a personal computer or a work station. The program can be distributed via a network, such as the Internet. The program can be recorded in a computer-readable and non-temporary recording medium, such as a hard disk, a flexible disk (FD), a CD-ROM, a MO, a DVD, or a Flash memory like a USB memory or a SD card, and can be read by a computer from the non-temporary recording medium and thus can be executed.

As described above, according to the embodiments, it is possible to increase image quality of an ultrasonic image while avoiding occurrence of artifact caused by a body motion.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A medical information processing apparatus (100) comprising:
an acquisition unit (111) configured to acquire a plurality of sets of ultrasonic data representing frames of a subject that are successive in a time direction,
a detection unit (112) configured to detect a motion of the subject,
a selection unit (113) configured to select, based on a result of detecting the motion, a plurality of sets of frame data representing subjects to be synthesized from the sets of ultrasonic data, and
a synthesizing process unit (114) configured to perform a synthesizing process on the selected sets of frame data.

2. The medical information processing apparatus (100) according to claim 1, wherein the synthesizing process unit (114) is configured to add the sets of frame data and generates addition data.

3. The medical information processing apparatus (100) according to claim 2, further comprising an extracting unit (115) configured to execute a process for high resolution of a subject that is presented by the addition data.

4. The medical information processing apparatus (100) according to claim 1, further comprising an extracting unit (115) configured to extract signal components representing a subject from the sets of frame data, respectively,
wherein the synthesizing process unit (114) is configured to perform a synthesizing process of generating integrated data obtained by integrating the signal components in the sets of frame data.

5. The medical information processing apparatus (100) according to claim 4, wherein the extracting unit (115) is configured to generate filter information corresponding to each position in the frame data based on a result of extracting signal components representing the subject in the sets of frame data, and
the synthesizing process unit (114) is configured to generate the integrated data using the filter information.

6. The medical information processing apparatus (100) according to claim 1, further comprising an extracting unit (115) configured to execute enhancing process of enhancing a subject on each of the sets of frame data,
wherein the synthesizing process unit (114) is configured to add the sets of frame data after the enhancing process and generate addition data.

7. The medical information processing apparatus (100) according to claim 1, wherein the detection unit (112) is configured to calculate an image quality evaluation index of each frame in the sets of ultrasonic data and, based on the image quality evaluation index, detect a motion of the subject.

8. The medical information processing apparatus (100) according to claim 1, wherein the detection unit (112) is configured to perform main component analysis on each frame in the sets of ultrasonic data and, based on a result of the analysis, detect a motion of the subject.

9. The medical information processing apparatus (100) according to claim 1, wherein the detection unit (112) is configured to detect a motion of the subject by inputting each frame in the sets of ultrasonic data to a trained model that is trained using a data set including ultrasonic data for training and information on presence or absence of a motion of a subject in the ultrasonic data for training.

10. The medical information processing apparatus (100) according to any one of claims 1 to 9, wherein the selection unit (113) is configured to select a plurality of sets of frame data with relatively a few motions of the subject from the sets of ultrasonic data as frame data representing frames that are the subjects to be synthesized.

11. The medical information processing apparatus (100) according to claim 10, wherein the selection unit (113) is configured to select, as the frame data representing frames that are the subjects to be synthesized, frame data on a side of a larger number of frames from the ultrasonic data of a set number of frames using a time at which a motion of the subject is detected by the detection unit (112) as a border.

12. The medical information processing apparatus (100) according to claim 10, wherein the selection unit (113) is configured to select, as the frame data representing frames that are the subjects to be synthesized, frame data that is acquired prior to a time at which a motion of the subject is detected by the detection unit (112) in the sets of ultrasonic data that are acquired over time.

13. The medical information processing apparatus (100) according to claim 10, wherein the selection unit (113) is configured to select, as the frame data representing frames that are the subjects to be synthesized, a plurality of sets of frame data having similarity at or above a reference between the frames represented by the sets of ultrasonic data.

14. A medical information processing method comprising:
an acquiring step of acquiring a plurality of sets of ultrasonic data representing frames of a subject that are successive in a time direction,
a detecting step of detecting a motion of the subject,
a selecting step of, based on a result of detecting the motion, selecting a plurality of sets of frame data representing subjects to be synthesized from the sets of ultrasonic data, and
a synthesizing process of performing a synthesizing process on the selected sets of frame data.
